(19) Europäisches Patentamt
European Patent Office
Office européen des brevets

(11) Veröffentlichungsnummer : **0 169 253**
**B1**

(12) # EUROPÄISCHE PATENTSCHRIFT

(45) Veröffentlichungstag der Patentschrift : **27.05.87**

(51) Int. Cl.⁴ : **C 07 C145/00**

(21) Anmeldenummer : **84108605.1**

(22) Anmeldetag : **20.07.84**

(54) **Verfahren zur Herstellung von Perchlormethylmercaptan.**

(43) Veröffentlichungstag der Anmeldung : **29.01.86 Patentblatt 86/05**

(45) Bekanntmachung des Hinweises auf die Patenterteilung : **27.05.87 Patentblatt 87/22**

(84) Benannte Vertragsstaaten : **AT BE DE FR GB IT NL SE**

(56) Entgegenhaltungen :
**DE-A- 2 156 329**
**FR-A- 1 437 908**

(73) Patentinhaber : **NITROKEMIA IPARTELEPEK**
**Pf. 45**
**H-8184 Füzfögyártelep (HU)**

(72) Erfinder : **Bakucz, Miklòs, Dipl.-Ing.**
**Május 1. tér 4**
**H-2536 Nyergesujfalu (HU)**
Erfinder : **Hornyák, Jòzsef, Dipl.-Ing.**
**Fadrusz u. 15**
**H-8220 Balatonalmádi (HU)**
Erfinder : **Nagy, Lajos, Dipl.-Ing.**
**Gagarin u. 1**
**H-8184 Balatonfüzfögyártelep (HU)**
Erfinder : **Pelyva, Jenö**
**Gagarin u. 2**
**H-8184 Balatonfüzfögyártelep (HU)**
Erfinder : **Sebök, Dezsö, Dr.**
**Halle u. 5/e**
**H-8200 Veszprém (HU)**
Erfinder : **Söptei, Csaba, Dipl.-Ing.**
**Festö u. 8**
**H-8200 Veszprem (HU)**
Erfinder : **Tömördi, Elemér, Dipl.-Ing.**
**Botev u. 4/b**
**H-8200 Veszprém (HU)**
Erfinder : **Vecsey Istváń K., Dipl.-Ing.**
**Gagarin u. 2**
**H-8184 Balatonfüzfögyártelep (HU)**
Erfinder : **Damján, János, Dipl.-Ing.**
**Gagarin u. 26**
**H-8184 Balatonfüzfögyártelep (HU)**

(74) Vertreter : **von Füner, Alexander, Dr. et al**
**Patentanwälte v. Füner, Ebbinghaus, Finck Mariahilfplatz 2 & 3**
**D-8000 München 90 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft ein verbessertes Verfahren zur Herstellung von Perchlormethylmercaptan.

Das Perchlormethylmercaptan — oder Trichlormethylsulfonylchlorid — ist ein wichtiges Zwischenprodukt, welches bei der Herstellung von fungiziden, bakteriziden und germiziden Mitteln, Schmierölzusatzstoffen, Bodendesinfektionsmitteln oder Arzneimitteln Verwendung finden kann.

Nach dem in Betrieb verbreitet verwendeten Verfahren wird Kohlenstoffdisulfid in wässrigem Medium chloriert, wobei als Zwischenprodukt als Folge einer oxydativen und hydrolytischen Zersetzung Schwefelsäure und Salzsäure gebildet werden, welche als Nebenprodukte verwendet werden können. Die Reaktion wird durch die folgende Gleichung illustriert :

$$CS_2 + 5\ Cl_2 + 4\ H_2O \longrightarrow ClS\text{—}CCl_3 + H_2SO_4 + 6\ HCl$$

Die Nebenprodukte können von dem gewünschten Produkt einfach getrennt werden. Es werden nämlich zwei Phasen — eine saure-wässrige Phase und eine, ausser dem erhaltenen Perchlormethylmercaptan auch das als Ausgangsstoff eingesetzte Kohlenstoffdisulfid und das als Folge einer Überchlorierung gebildete Kohlenstofftetrachlorid enthaltende organische Phase — gebildet. Die letzteren Verbindungen können vom Perchlormethylmercaptan auf Grund des großen Siedepunktunterschiedes getrennt werden.

Die Herstellung von Perchlormethylmercaptan in wässrigem Medium wird auch in der französischen Patentschrift Nr. 1 437 908 beschrieben. Hier wird die Rolle und Wichtigkeit der Salzsäure bei der Chlorierung des Kohlenstoffdisulfids in wässrigem Medium betont. Die Geschwindigkeit der Chlorierung und die Ausbeute hängen von der Anfangskonzentration der Salzsäure ab. Als optimale Salzsäurekonzentration wird das Interval von 15-20 % bezeichnet. Mehrwertige Metalle (z. B. Blei, Cobalt, Nickel usw.) und Emulgierungsmittel erhöhen die Reaktionsgeschwindigkeit. Die Ausbeute beträgt 88 %, die Reinheit des Produktes ist 95 %.

In der US Patentschrift Nr. 3 993 693 ist ein kontinuierliches Verfahren beschrieben. Nach diesem Verfahren ist die Anfangskonzentration der Schwefelsäure 40 %, die Verweilzeit im Reaktor beträgt 3,5 Stunden. Es wird eine Ausbeute von 78,4 % erreicht. Die wässrige Phase wird durch Destillation zu Schwefelsäure und Salzsäure getrennt.

Nach der DPS Nr. 2 156 329 werden gemäß einem kontinuierlichen Verfahren in einen 3 m langen Rohrreaktor Wasser, Kohlenstoffdisulfid und Chlor kontinuierlich eingeführt. Die Ausbeute beträgt 89,5 %, die Raum-Zeit-Ausbeute ist 28,8 kg/100 l/Stunde.

Nach den bekannten Verfahren wurde der folgende Zusammenhang zwischen der Erhöhung der Rekationstemperatur und der Ausbeute festgestellt : bis 30 °C wird die Reaktionsgeschwindigkeit größer, über 30 °C wird die Reaktion langsamer und über 40 °C findet praktisch keine Perchlormethylmercaptanbildung mehr statt. Aus diesem Grunde wird die Chlorierung bei niedriger Temperatur durchgeführt. Die Verwirklichung der Umsetzung ist deshalb kompliziert, weil die im Laufe der Reaktion gebildete Wärmemenge den üblichen Wert übertrifft (160 Kal/Mol). Die gebildete große Wärmemenge muß bei niedriger Temperatur abgeführt werden, was die Ausbildung und Inbetriebhaltung eines Kühlsystems und eine proportionale Vergrößerung der eingebauten Kühloberfläche fordert. Die Wärmeabfuhr ist auch mit Schwierigkeiten verbunden, weil das Medium korrosiv ist und die anwendbaren Konstruktionsmaterialien eine geringe Wärmeleitfähigkeit besitzen ; deswegen müssen größere Einrichtungen eingesetzt werden. Eine weitere Schwierigkeit liegt darin, daß große Volumina von sehr feuergefährlichen und gesundheitsschädlichen Materialien gleichzeitig aufgearbeitet und behandelt werden müssen.

Neben der Hauptreaktion findet auch eine Überchlorierung statt. Die Menge des so gebildeten Kohlenstofftetrachlorids ist mid dem Konversionsgrad proportional. Um eine Überchlorierung zu vermeiden wird die Chlorierung nur bis zu einem niedrigen Chlorierungsgrad durchgeführt und ein Teil des Kohlenstoffdisulfids wird in die Reaktion zurückgeführt.

Das unreagierte Kohlenstoffdisulfid wird durch Destillation zurückgewonnen. Bei der Destillation bzw. Vakuumdestillation stellt die Gegenwart von Luft wegen der niedrigen Selbsentzündungstemperatur (98 °C) eine potentielle Feuergefahr dar.

Das Ziel der Erfindung ist die Schaffung eines verbesserten Verfahrens zur Herstellung von Perchlormethylmercaptan und die Behebung der obigen Nachteile der bekannten Verfahren.

Die Erfindung beruht auf der Erkenntnis, daß die Geschwindigkeit der Chlorierung von Kohlenstoffdisulfid in wässrigem Medium unter bestimmten Bedingungen proportional zur Steigerung der Temperatur erhöht wird. Die Geschwindigkeit wird durch einen physikalischen und einen chemischen Vorgang beeinflusst. Der physikalische Vorgang ist die Lösbarkeit des Chlorgases in der wässrigen und organischen Phase. Der chemische Vorgang ist die durchzuführende Reaktion. Bei einer Temperatur über 30 °C wird die Löslichkeit des Chlorgases in wässrigem Medium geringer, was eine Herabsetzung der Reaktionsgeschwindigkeit zur Folge hat. Es wurde gefunden, daß bei Erhöhung der Reaktionstemperatur die Reaktionsgeschwindigkeit deshalb herabgesetzt wird, weil das Chlorgas in wässrigem Medium nicht mehr lösbar ist. Falls es gelingt die Löslichkeit aufrechtzuerhalten, wird die Reaktionsgeschwindigkeit bei Erhöhung der Temperatur größer. Mit Rücksicht darauf, daß bei der Umsetzung die Berührung von drei verschiedenen Phasen bzw. die Umsetzung von in drei verschiedenen Phasen anwesenden Substanzen notwendig ist, muß zwecks besserer Berührung der Reaktionskomponenten eine spezielle Umrührung verwendet werden. Dies kann mit Hilfe von mechanischen Rührern, Turbinenrührern, Zentrifugalpumpen, Vibratorrührern, in Rohren durch turbulente Strömung, Ultraschallerregern oder anderen ahnlichen Einrichtungen bzw. Kombinationen davon durchgeführt werden.

Es ist zweckmäßig das Verfahren in einem kontinuierlichen System durchzuführen, worin das Material in einer geeigneten Einrichtung kontinuierlich vorwärtsdringt und dabei eine intensive effektive Vermischung stattfindet. Anstatt der intensiven Umrührung kann ein Überdruck oder eine Komination der beiden Maßnahmen verwendet werden.

Gegenstand der Erfindung ist ein Verfahren zur Herstellung von Perchlormethylmercaptan, durch Chlorierung vom Kohlenstoffdisulfid in wärrigem Medium, dadurch gekennzeichnet, daß man die Ausgangsstoffe und das Chlorgas unter atmosphärischem oder erhöhtem Druck, bei einer Temperatur über dem Siedepunkt des Kohlenstoffdisulfids in das, in ständigem Umlauf gehaltene, schwefelsäuresalzsäure-haltige wässrige Katalysatormedium einführt ; die Reaktionspartner durch Steigerung der Intensität der Rührung oder Anwendung eines erhöhten Druckes in wässrigem Medium adsorbiert ; nach Ablauf der Reaktion aus dem Reaktionsgemisch die organische Phase abtrennt und zweckmäßig durch Wasserdampfdestillation fraktioniert und das wässrige Medium als Katalysator in den Reaktionsvorlauf zurückführt.

Mit Rücksicht darauf, daß nach dem erfindungsgemäßen Verfahren im Verhältnis zu den bekannten Methoden bei erhöhter Temperatur gearbeitet wird, wird die Reaktionsgeschwindigkeit größer und die Verweilzeit im Reaktor kürzer. Das kontinuierliche Verfahren kann deshalb auch in kleineren Apparaten durchgeführt werden. Unter Anwendung von kleineren Reaktoren ist eine geringere Materialmenge im Apparat anwesend. Da die Reaktion bei höherer Temperatur durchgeführt wird, kann zur Kühlung auch Betriebswasser dienen und die Einschaltung von besonderen Kühleinrichtungen erübrigt sich. Wegen der größeren Reaktionsgeschwindigkeit muß nur eine geringere Menge von feuergefährlichen Substanzen gleichzeitig behandelt werden, so daß die Feuer- und Gesundheitsprobleme verringert werden.

Ein weiterer Vorteil der erhöhten Reaktionsgeschwindigkeit besteht darin, daß die Verweilzeit der Reaktionspartner im System kurz ist, so daß das Risiko einer Überchlorierung auf das Minimum vermindert wird.

Weitere Einzelheiten des erfindungsgemäßen Verfahrens sind den nachstehenden Beispielen zu entnehmen ohne den Schutzumfang auf diese Beispiele einzuschränken.

## Beispiel 1 (Vergleichsbeispiel)

In einen 1,5 l großen, mit einem intensiven Saugrührer versehenen Kolben werden 76 g Kohlenstoffdisulfid, 570 ml Wasser und 500 ml einer, 12 % Schwefelsäure und 26,7 % Salzsäure enthaltenden sauren Lösung eingewogen. Der Kolben ist mit einem, unter das Flüssigkeitsniveau hereinreichenden Gaseinleitungsrohr ausgerüstet. Auf das Entlüftungsrohr des sehr sorgfältig verschlossenen Kolbens wird zwecks Nachweis der den Kolben verlassenden nicht-reagierten Gase ein mit Wasser gefüllter Blasenbildner installiert. Durch den mit einer hohen Umdrehungszahl operierenden Saugrührer werden aus dem Gasraum des Kolbens die Gasblasen in die Flüssigkeit zurückgetragen und dadurch wird eine intensive Berührung zwischen der Gas- und Flüssigkeitsphase zu Stande gebracht. Eine stöchiometrische berechnete Menge (354,5 g) von Chlorgas wird unter Kühlung mit einer solchen Geschwindigkeit eingeleitet, daß kein bedeutender Chlorgasdurchbruch stattfinden soll. Die Temperatur des Reaktionsgemisches wird durch Kühlung unter 30 °C gehalten. Die notwendige Chlormenge kann innerhalb von etwa 3 Stunden eingeleitet werden. Am Ende der Reaktion und nach Beendigung der Rührung bildet das Reaktionsgemisch zwei Phasen.

Als untere Phase werden 165 g einer organischen Schicht abgetrennt (Perchlormethylmercaptangehalt 91,2 %). Ausbeute 81 %. Das erhaltene Produkt enthält 3,1 % unreagiertes Kohlendisulfid, 5,3 % des aus der Überchlorierung stammenden Kohlenstofftetrachlorids und 0,4 % andere Verunreinigungen.

## Beispiel 2

In den im Beispiel 1 beschriebenen Apparat werden 76 g Kohlenstoffdisulfid, 325 ml Wasser und 750 ml einer, 12 % Schwefelsäure und 26,7 % Salzsäure enthaltenden sauren Lösung eingewogen. Unter Kühlung werden bei 30 °C innerhalb von 50 Minuten 200 g Chlorgas eingeleitet. Nach Phasenabtrennung werden 127 g (73,92 %) Perchlormethylmercaptan, 24,98 % Kohlenstoffdisulfid und 0,76 g Kohlenstofftetrachlorid erhalten. Die auf das Chlor bezogene Ausbeute beträgt 89,55 %. Durch die partielle Chlorierung kann die Ausbeute verbessert, die aus der Überchlorierung stammende Kohlenstofftetrachloridmenge vermindert und die Geschwindigkeit der Perchlormethylmercaptanbildung auf das etwa 2,2-Fache erhöht werden. Das chlorierte Rohgemisch wird durch Wasserdampfdestillation aufgearbeitet. Bei dem Wasserdampfdestillation werden 36 g eines Vorlaufes (Kohlenstoffdisulfidgehalt) (85 %) und 86 g eines Perchlormethylmercaptanhauptdestillats erhalten. Der Perchlormethylmercaptangehalt des Hauptdestillats beträgt 98,2 %.

Beispiel 3

In Gasausleitungsrohr des Apparats nach Beispiel 1 wird ein sich bei 50 KPa öffnendes Ventil installiert. In den Apparat werden 76 g Kohlenstoffdisulfid, 325 ml Wasser und 750 ml einer, 12 % Schwefelsäure und 26,7 % Salzsäure enthaltenden sauren Lösung eingewogen. Bei einer Reaktionstemperatur von 35 °C und unter einem Überdruck von 50 KPa werden innerhalb von 28 Minuten 200 g Chlor umgesetzt. Das erhaltene Reaktionsprodukt enthält 125 g Perchlormethylmercaptan (73,1 %), 25,8 % Kohlenstoffdisulfid und 0,78 % Kohlenstofftetrachlorid. Ausbeute 87,2 % (auf das eingeleitete Chlor bezogen). Die Reaktionsgeschwindigkeit kann dadurch erhöht werden, daß man die Chlorierung unter Druck durchführt. Das Reaktionsgemisch kann auf die oben beschriebene Weise durch periodische Wasserdampfdestillation aufgearbeitet werden.

Beispiel 4

Die kontinuierliche Chlorierung wird in einen, mit einem Rührer versehenen, in mehrere Kammer aufgeteilten Reaktor (Länge 2 m, Durchmesser 200 mm) durchgeführt. 13,0 kg/Stunde Kohlenstoffdisulfid, 42,5 kg/Stunde Chlor, 69 kg/Stunde Wasser und als Katalysator 400 kg/Stunde einer, 12 % Schwefelsäure und 26,7 % Salzsäure enthaltenden sauren wässrigen Lösung werden in den unteren Teil des Reaktors eingeleitet. Das den Reaktor verlassende Gemisch von saurem Wasser und einer organischen Phase wird auf einem Separator getrennt. Der Hauptteil der Säure wird nach Erwärmen in den Reaktor als Katalysator zurückgeführt. Die den Reaktor verlassende organische Phase (24,95 kg/Stunde) enthält 83,2 % Perchlormethylmercaptan, 15 % Kohlenstoffdisulfid, 1,15 % Kohlenstofftetrachlorid und 0,65 % andere Verunreinigungen. Das chlorierte Reaktionsgemisch wird bei der Mitte einer Rektifizierungskolonne eingeführt. Der Kohlenstoffdisulfidgehalt des Produktes wird mit Hilfe des am unteren Teil der Kolonne zugegebenen Wasserdampfes und des am Kopfteil gebildeten Rückflusses entfernt. Es werden 19,95 kg/Stunde Perchlormethylmercaptan erhalten (Reinheitsgrad : 94 %). Das durch Destillation entfernte Kohlenstoffdisulfid wird am Anfang des Vorganges zurückgeführt.

**Patentanspruch**

Verfahren zur Herstellung von Perchlormethylmercaptan, durch Chlorierung vom Kohlenstoffdisulfid in wässrigem Medium dadurch gekennzeichnet, daß man die Ausgangsstoffe und das Chlorgas unter atmosphärischem oder erhöhtem Druck, bei einer Temperatur über dem Siedepunkt des Kohlenstoffdisulfids in das, in ständigem Umlauf gehaltene, schwefelsäuresalzsäure-haltige wässrige Katalysatormedium einführt ; die Reaktionspartner durch Steigerung der Intensität der Rührung oder Anwendung eines erhöhten Druckes in wässrigem Medium adsorbiert ; nach Ablauf der Reaktion aus dem Reaktionsgemisch die organische Phase abtrennt und zweckmäßig durch Wasserdampfdestillation fraktionniert und das wässrige Medium als Katalysator in den Reaktionsvorlauf zurückführt.

**Claim**

Process for preparing perchloromethylmercaptan by chlorination of carbon disulphide in an aqueous medium, characterised in that the starting materials and the chlorine gas are introduced, under atmospheric or elevated pressure, at a temperature above the boiling point of the carbon disulphide, into the aqueous catalyst medium which contains sulphuric and hydrochloric acids and is kept constantly circulating ; the reactants are adsorbed in the aqueous medium by increasing the intensity of stirring or by applying elevated pressure ; and after the reaction has ended the organic phase is separated out from the reaction mixture and conveniently fractionated by steam distillation and the aqueous medium is recycled into the reaction process as catalyst.

**Revendication**

Procédé de préparation de perchlorométhylmercaptan, par chloruration de disulfure de carbone en milieu aqueux, caractérisé en ce qu'on introduit les produits de départ et le chlore gazeux à une pression égale ou supérieure à la pression atmosphérique, à une température supérieure au point d'ébullition du disulfure de carbone dans le milieu catalyseur aqueux contenant de l'acide sulfurique-acide chlorhydrique, maintenu en rotation constante ; en ce qu'on adsorbe les réactifs en accroissant l'intensité de l'agitation ou en appliquant une pression augmentée en milieux aqueux ; après la fin de la réaction en ce qu'on sépare du mélange réactionnel la phase organique et en ce qu'on fractionne en pratique par distillation à la vapeur d'eau et en ce qu'on réintroduit le milieu aqueux comme catalyseur dans le cours de la réaction.